# EUROPEAN PATENT APPLICATION

(11) **EP 4 582 046 A1**
(43) Date of publication of application: **09.07.2025**
(21) Application number: 22958050.1
(22) Date of filing: 05.09.2022
(51) Int. Cl.: A61B 34/37

(54) **MEDICAL DEVICE**

(71) Applicant: RIVERFIELD Inc., Tokyo 107-0052 (JP)
(72) Inventor: SUZUKI, Masataka, Tokyo 107-0052 (JP)
(74) Representative: Adares PartGmbB
(86) International application number: PCT/JP2022/033321
(87) International publication number: WO 2024/052969

(57) **Abstract**

Provided is a medical device that can efficiently perform an accurate alignment between a medical instrument and a patient. The medical device according to the present disclosure arranges a pivot movement point of the medical instrument at a desired location on a body surface of the patient. The medical device includes a base portion, a connecting portion, a distal portion, and a support portion that is connected to the base portion and to the distal portion and that is configured to change an orientation of the medical instrument to a desired orientation around the pivot movement point as a center of rotation. The distal portion further includes a first light emitter configured to emit light in a preset direction when the pivot movement point is arranged.

## Description

### TECHNICAL FIELD

The present disclosure relates to a medical device in which a specific portion of a medical instrument connected to the medical device is arranged at a desired location on a patient, and the medical instrument is supported in a desired orientation.

### BACKGROUND ART

Laparoscopic surgery using a master-slave type surgical robot system has become widespread. Typically, the surgical robot system includes a patient-side unit to be arranged near a patient, and an operation unit that a surgeon operates to perform surgery.

In laparoscopic surgery using such a surgical robot system, a medical instrument having an elongated shaft shape, such as an endoscope and forceps, is connected to a manipulator arm of the patient-side unit, and is used. A distal part of the connected medical instrument is inserted into the patient's abdominal cavity via a trocar provided by penetrating the abdominal wall.

The surgeon operates the operation unit to cause the manipulator arm of the patient-side unit or the medical instrument connected to the manipulator arm to perform movement corresponding to the operation, thereby performing a desired treatment on a patient's target site. In accordance with the operation performed by the surgeon, the manipulator arm changes its orientation, thereby changing a position, an angle or the like of the medical instrument connected to the manipulator arm relative to the patient.

Since the medical instrument connected to the manipulator arm is inserted into the patient's abdominal cavity via the trocar, a range where the medical instrument can move is limited to a specific range. In addition, the angle of the medical instrument relative to the patient is limited to a specific range.

Such limitations are caused by, for example, the position of the medical instrument relative to an arrangement position of the trocar provided on the patient, by the position of the manipulator arm relative to the patient, by a range of motion of the manipulator arm, and the like.

Accordingly, it is necessary to appropriately perform alignment between the manipulator arm, the medical instrument connected to the manipulator arm, and the trocar prior to surgery. That is, before starting surgery using the surgical robot system, each of the relative positions of the medical instrument relative to an arrangement position of the trocar arranged on the patient, the manipulator arm relative to the patient, and the like needs to be adjusted to achieve an appropriate alignment relationship.

Typically, in the laparoscopic surgery using the surgical robot system, the above-described alignment is performed using a light source for alignment. For example, a laser light source for alignment is used to emit light, such as laser light, to a position where the trocar is to be arranged, thereby performing alignment between the patient and the surgical robot system.

For example, Patent Document 1 discloses a medical manipulator system including an alignment device for performing alignment between a manipulator arm that supports a surgical tool such as an endoscope and forceps in a desired orientation, and a trocar into which the surgical tool is inserted. **In** this medical manipulator system, a link mechanism provided at a distal side of an arm is driven, thereby changing a position of the surgical tool connected to the medical manipulator system and an angle of the surgical tool relative to the patient. The alignment device is used to perform alignment between an immovable point of the link mechanism and a position of the trocar. The alignment device is provided with a light source portion (light emitter) that emits light to the immovable point of the link mechanism. A worker, who performs alignment, performs alignment between the immovable point of the link mechanism and the position of the trocar, using the light emitted from the light source portion as a reference.

Furthermore, Patent Document 2 discloses a teleoperational medical system (surgical robot system) equipped with a guided setup system for guiding a preoperative preparation. Patent Document 2 discloses that light such as laser light is used in the guided setup system.

### PRIOR ART DOCUMENTS

### PATENT DOCUMENTS

Patent Document 1: Japanese Unexamined Patent Application Publication No. 2019-98496
Patent Document 2: Japanese Patent No. 6725424

### SUMMARY OF THE INVENTION

### PROBLEMS TO BE SOLVED BY THE INVENTION

In the conventional technique described above, since a light source portion is located away from the surgical tool, there are cases where it is difficult to perform an efficient and accurate alignment. For example, the light source portion in the technique disclosed in Patent Document 1 or Patent Document 2 is placed away from an alignment target site onto which light is projected, and thus a portion of the link mechanism, a portion of a worker's body, and the like may be situated between the alignment target site and the light source portion, thus blocking light emitted from the light source portion. In addition, in the technique described in Patent Document 1, the light source portion is provided at the distal part of an arm away from the surgical tool. This may cause a position of the light emitted to the alignment target site to be significantly displaced even if the arm is slightly moved.

The present disclosure discloses one example of a medical device that can efficiently perform an accurate alignment between a medical instrument and a patient.

### MEANS FOR SOLVING THE PROBLEMS

A medical device according to the present disclosure in which a pivot movement point of a medical instrument connected to the medical device is arranged at a desired location on a body surface of a patient comprises a base portion, a connecting portion to which the medical instrument is to be connected, a distal portion including the connecting portion, and a support portion connected to each of the base portion and the distal portion and configured to change an orientation of the medical instrument to a desired orientation around the pivot movement point as a center of rotation. The distal portion further includes a first light emitter configured to emit light in a preset direction when the pivot movement point is arranged.

According to the medical device of the present disclosure, when the pivot movement point of the medical instrument connected to the medical device is arranged at the desired location on the patient's body surface, the first light emitter emits light in the preset direction. This makes it easier to perform an adjustment so that the pivot movement point, which serves as the center of rotation, is arranged at the patient's desired location. Hereinafter, adjusting the orientation or a position of the medical device, a position of the patient, or the like to arrange a specific portion of the medical instrument at the patient's desired location is also referred to as "alignment between the patient and the medical device", alternatively, simply referred to as "alignment". According to the medical device of the present disclosure, since it is possible to perform alignment based on a projection position of light from the first light emitter as a reference, an assistant (hereinafter, also referred to as a worker) in surgery, who performs the alignment between the patient and the medical device, can easily perform a work of alignment.

Furthermore, since the first light emitter is arranged at the distal portion including the connecting portion to which the medical instrument is to be connected, it is easier to perform the work of alignment. That is, since a distance between the patient's desired location and the first light emitter is short, it is easier to perform the work of alignment. **In** addition, when the alignment is performed, a portion of the body of the worker who is performing the alignment or a portion of the support portion does not tend to be situated between the first light emitter and the patient. That is, the portion of the worker's body or the portion of the support portion does not tend to block the light from the first light emitter during the work of alignment. Accordingly, the worker can efficiently perform the alignment between the patient and the medical device.

**In** the above configuration, it is preferable that the first light emitter includes a first light source and a second light source, and that the first light source and the second light source are arranged such that a first optical axis of the first light source and a second optical axis of the second light source intersect at a specific relative position from the first light source and the second light source.

With this configuration, an accurate alignment between the patient and the medical device can be performed. That is, a site where the projected image of the light from the first light source and the projected image of the light from the second light source are projected and overlap each other is located at the patient's desired location, and this makes it possible to perform an accurate alignment in a direction (light emission direction) to which the light emitted from the first light emitter directs. For example, when the first light source and the second light source are arranged such that a first optical axis of the first light source and a second optical axis of the second light source intersect at the pivot movement point, the worker can precisely arrange the pivot movement point at the patient's desired location based on the position where the first optical axis and the second optical axis intersect as a reference, and thus an accurate arrangement can be ensured. In other words, it is possible to perform an accurate alignment between the patient's desired location and the pivot movement point of the medical instrument in the light emission direction of the first light emitter.

In the above configuration, it is preferable that the first light source and the second light source are aligned in a direction intersecting a direction extending from the base portion toward the distal portion.

With this configuration, it is easier to perform the work of alignment by moving the distal portion in a direction away from the base portion or bringing it in a direction closer to the base portion. That is, when the worker performs the alignment by moving the distal portion in the direction away from the base portion or bringing it in the direction closer to the base portion, it is easier to perform the work of alignment so that the site onto which the light from the first light source and the light from the second light source are projected and overlap each other is located at the patient's desired location.

In the above configuration, it is preferable that at least one of the first light source or the second light source is configured such that a projected light has a cross shape.

With this configuration, the worker can easily recognize the site where the first optical axis and the second optical axis intersect. **In** other words, the worker can easily perform the work of alignment so that the patient's desired location is located at the site where the first optical axis and the second optical axis intersect. For example, in a case in which the projected shape of the light from the first light source is configured to have a cross shape, the worker can compare a center position of the cross-shaped projected image of the light from the first light source and a center position of the projected image of the light from the second light source with each other, thereby grasping an extent of a positional misalignment and its direction. For example, in a case in which the projected image of the light from the second light source is configured to have a cross shape, the worker can compare the center position of the cross-shaped projected image of the light from the second light source and the center position of the projected image of the light from the first light source with each other, thereby grasping an extent of a positional misalignment and its direction. Accordingly, the worker can easily perform an accurate alignment by overlapping the respective centers of the lights of the projected images. **In a** case in which the projected shape of the light from the first light source and the projected image of the light from the second light source are each configured to have a cross shape, the worker can easily perform an accurate alignment by overlapping the center position of the cross-shaped projected image of the light from the first light source and the center position of the cross-shaped projected image of the light from the second light source at the same position.

**In** the above configuration, it is preferable that the medical device further comprise a cart portion that is movable and to which the base portion is mounted, wherein the base portion includes a second light emitter configured to emit light toward a floor on which the cart portion is arranged.

With this configuration, the light emitted from the second light emitter is projected onto a floor where the cart portion is arranged. The worker checks a position of the light projected onto the floor, thereby easily grasping the relationship between the position of the patient and the position of the medical device. In addition, the worker can move the cart portion, thereby adjusting the position of the medical device relative to the patient.

In the above configuration, it is preferable that the support portion is configured to change a position of the distal portion such that a distance between the first light emitter and the second light emitter is a specific distance.

With this configuration, the distance between the first light emitter and the second light emitter is kept constant. In other words, the support portion changes the position of the distal portion so that the distance between the distal portion and the base portion is a specific distance. The distance between the distal portion and the base portion is kept at the specific distance, thereby inhibiting a range of motion of the support portion from being limited. For example, an unnecessary restriction of the range of motion of the support portion due to a short distance between the distal portion and the base portion is inhibited.

**In** the above configuration, it is preferable that the first light emitter is configured to emit light of a color complementary to red and/or a color opposite to red.

With this configuration, if the light from the first light emitter is projected onto a portion of the patient covered in the patient's blood, the worker can easily recognize the position of the projected light. That is, even if a desired portion of the patient is covered in the blood, the worker can accurately perform the work of alignment between the patient and the medical device. In addition, the work of alignment between the patient and the medical device can be easily performed.

In the above configuration, it is preferable that the second light emitter is configured to emit light of a color complementary to green and/or a color opposite to green.

Typically, floors in surgery rooms are colored green or made of green floor materials. Accordingly, when the second light emitter emits light of a color complementary to green and/or a color opposite to green, the worker can easily recognize the light projected onto the green floor. That is, the worker can accurately perform the work of alignment between the patient and the medical device. In addition, the work of alignment between the patient and the medical device can be easily performed.

### EFFECTS OF THE INVENTION

According to the present disclosure, it is possible to provide a medical device in which an accurate alignment between a medical instrument and a position of a patient can be easily performed.

### BRIEF DESCRIPTION OF THE DRAWINGS

FIG. 1 is an external view illustrating a medical device according to the present disclosure.
FIG. 2 is a diagram illustrating a first light emitter of the medical device according to the present disclosure.
FIG. 3 is a diagram illustrating a second light emitter of the medical device according to the present disclosure.
FIG. 4 is a diagram illustrating a state in which a medical instrument is connected to the medical device according to the present disclosure.
FIG. 5 is a diagram illustrating a relationship between light emitted from a first light source and light emitted from a second light source, according to the present disclosure.
FIG. 6 is a diagram illustrating a state in which alignment is properly performed.
FIG. 7 is a diagram illustrating one example state in which the alignment is not properly performed.
FIG. 8 is a diagram illustrating another example state in which the alignment is not properly performed.
FIG. 9 is a diagram illustrating a relationship between the first light emitter and the second light emitter, according to the present disclosure.

### EXPLANATION OF REFERENCE NUMERALS

1...surgical robot system, 2...operation unit, 3...controller, 10...patient-side unit (medical device), 12a, 12b, 12c...distal portions, 13a, 13b, 13c...manipulator arms, 14... base portion, 15...base support, 16...cart portion, 17a, 17b, 17c...floor-side surfaces, 21a, 21b, 21c...joints, 30a... first light emitter, 31a...first light source, 31b...second light source, 33a...first optical axis, 33b...second optical axis, 34...intersection point, 35a, 35b...projected shapes, 41a...rigid endoscope (medical instrument), 41b, 41c...surgical instruments (medical instruments), 42a, 42b, 42c...connecting portions, 45...pivot movement point, 50...patient, 51...abdominal cavity, 52...trocar, 53...through-hole, 54...desired location, 60...second light emitter, 61...lower surface, 80...floor

### MODE FOR CARRYING OUT THE INVENTION

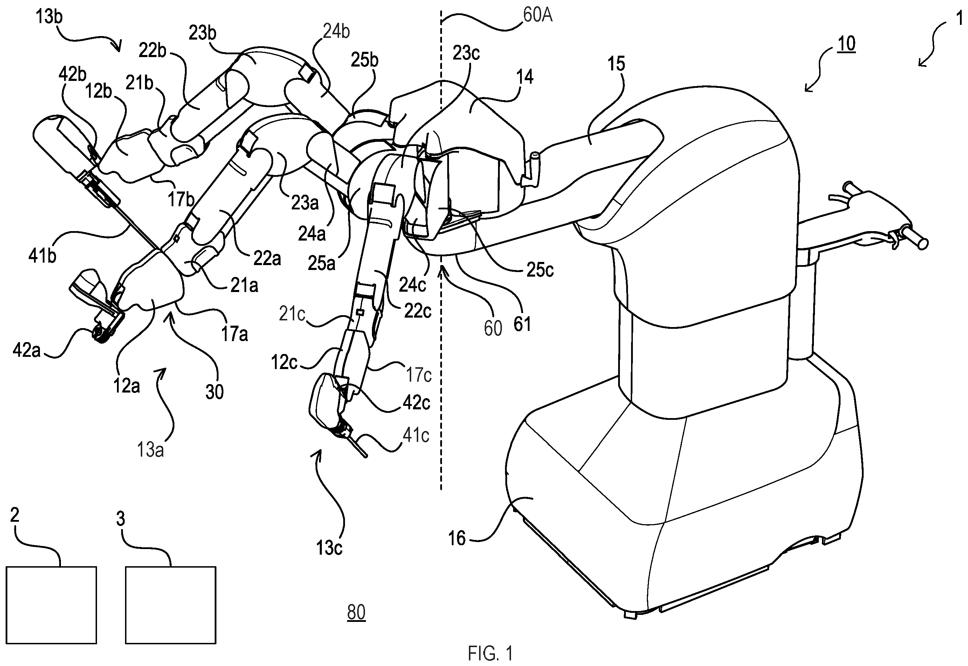

A medical device of one embodiment according to the present disclosure will be described with reference to FIG. 1 through FIG. 9. Arrows and diagonal lines indicating directions in each figure are illustrated to facilitate understanding relationships between the figures, as well as the shape of each component or portion. Accordingly, the technical content described in the present disclosure is not limited by directions illustrated in the drawings. The drawings with hatched lines provided thereon are not necessarily sectional views.

The medical device according to the present disclosure is used as a slave device in a master-slave type surgical robot system 1. The surgical robot system 1 of the present embodiment includes a controller 3, a patient-side unit 10 that is positioned near the patient and that performs a desired treatment on the patient, and an operation unit 2 operated by a surgeon performing surgery. Hereinafter, the patient-side unit 10 may also be referred to as a medical device 10.

The operation unit 2 is an operation device operated by the surgeon performing surgery. The controller 3 is a portion that controls the medical device 10 primarily in response to operations performed on the operation unit 2. The controller 3 outputs an operation signal in accordance with the operation that has been performed on the operation unit 2, and controls manipulator arms 13a, 13b, 13c.

The controller 3 further has a function of switching operation modes of the medical device 10. The operation modes of the medical device 10 include an operating mode in which movement is performed in response to the operation signal and a setting mode in which setting is performed by an assistant located near the patient. When the operation mode of the medical device 10 is switched to the setting mode, the medical device 10 is controlled such that the assistant (worker) can manually change arrangement positions of the manipulator arms 13a, 13b, 13c. That is, the assistant applies force to specific portions of the manipulator arms 13a, 13b, 13c, thereby allowing the medical device 10 to change the arrangement positions of the manipulator arms 13a, 13b, 13c without needing to operate the operation unit 2.

In FIG. 1, the controller 3 is described as a separate unit for illustrative purposes, but the operation unit 2 may have a function as the controller 3. Alternatively, the medical device 10 may have the function as the controller 3. Alternatively, the medical device 10 and the operation unit 2 may each have the function as the controller 3.

### 1. Description of Configuration

As shown in FIG. 1, the medical device 10 includes the manipulator arms 13a, 13b, 13c, a base portion 14, distal portions 12a, 12b, 12c, connecting portions 42a, 42b, 42c, and a first light emitter 30a. The medical device 10 further includes a cart portion 16 and a second light emitter 60. Hereinafter, sides of the manipulator arms 13a, 13b, 13c adjacent to the base portion 14 are also referred to as "proximate sides", and the opposite sides are also referred to as "distal sides". The medical device 10 may include two manipulator arms or fewer than two manipulator arms. Alternatively, the medical device 10 may include four manipulator arms or more than four manipulator arms.

As shown in FIG. 1, the manipulator arm 13a is a multi-joint arm comprising column-shaped arms 22a, 24a and joint members 21a, 23a, 25a. The arms 22a, 24a are connected to each other by the joint member 23a arranged therebetween such that the arms 22a, 24a are each rotatable. The proximate side of the arm 24a is rotatably connected to the base portion 14 by the joint member 25a. The distal side of the arm 22a is rotatably connected to the distal portion 12a via the joint member 21a.

The joint members 21a, 23a, 25a are each provided with an actuator (not shown). In the manipulator arm 13a, each actuator operates in response to the corresponding operation signal from the controller 3, and the positions of the arms 22a, 24a relative to the base portion 14 are changed. That is, each actuator operates in response to the corresponding operation signal from the controller 3, and the orientation of the manipulator arm 13a is changed. That is, arrangement positions of the arms 22a, 24a, an angle between the arm 22a and the arm 24a, and respective angles of the arms 22a, 24a to the base portion 14 or to the distal portion 12a are changed. The orientation of the manipulator arm 13a is changed in response to the operation signal from the controller 3, thereby moving the distal portion 12a in a desired direction.

The manipulator arms 13b, 13c are each configured in the same manner as the manipulator arm 13a. As shown in FIG. 1, the manipulator arm 13b comprises column-shaped arms 22b, 24b and joint members 21b, 23b, 25b. A proximate side of the manipulator arm 13b is connected to the base portion 14 by the joint member 25b such that the manipulator arm 13b is rotatable relative to the base portion 14. Also, at a distal side of the manipulator arm 13b, the distal portion 12b is rotatably connected to the arm 22b via the joint member 21b.

The joint members 21b, 23b, 25b are each provided with an actuator (not shown). In the same manner as the manipulator arm 13a, each actuator operates in response to the corresponding operation signal from the controller 3 to change the orientation of the manipulator arm 13b.

As shown in FIG. 1, the manipulator arm 13c comprises column-shaped arms 22c, 24c and joint members 21c, 23c, 25c. A proximate side of the manipulator arm 13c is connected to the base portion 14 by the joint member 25c such that the manipulator arm 13c is rotatable relative to the base portion 14. Also, at a distal side of the manipulator arm 13c, the distal portion 12c is rotatably connected to the arm 22c via the joint member 21c.

The joint members 21c, 23c, 25c are each provided with an actuator (not shown). In the same manner as the manipulator arm 13a, each actuator operates in response to the corresponding operation signal from the controller 3 to change the orientation of the manipulator arm 13c. The manipulator arms 13a, 13b, 13c are examples of support portions. The manipulator arms 13a, 13b, 13c are each not limited to the above-described configuration, as long as a desired operation is performed in response to the operation signal.

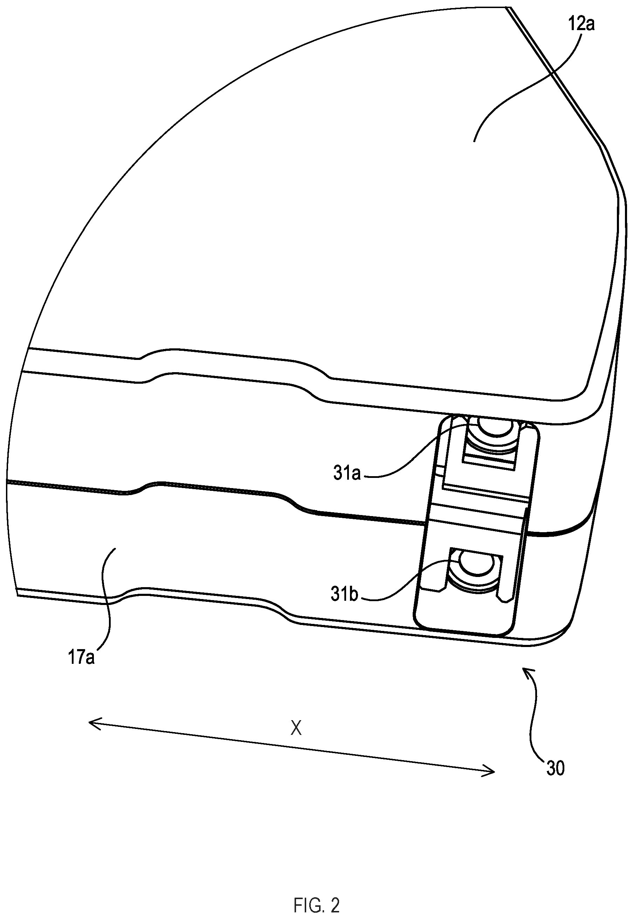

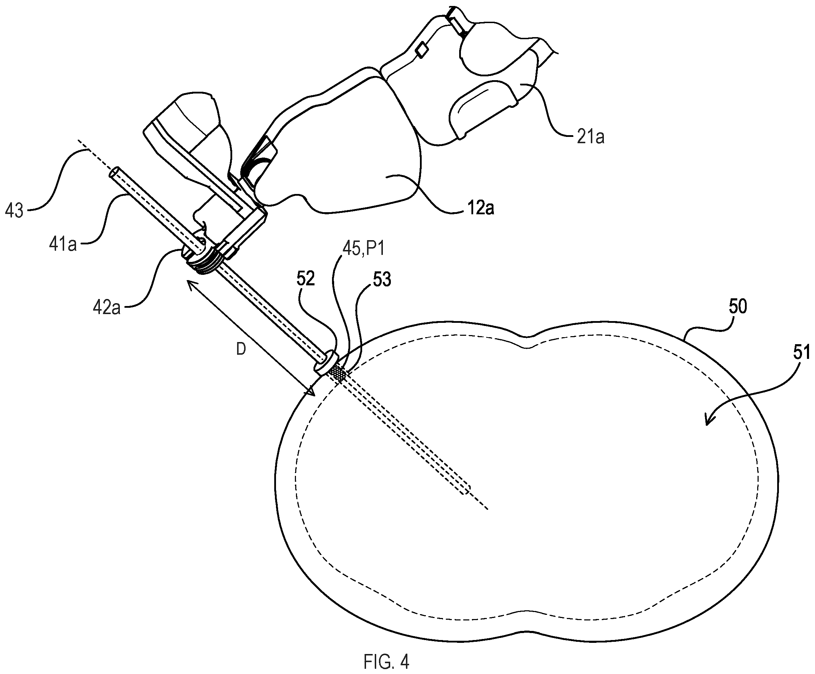

The distal portion 12a includes the connecting portion 42a. As shown in FIG. 4, a medical instrument 41a having a shaft shape is connected to the connecting portion 42a. An example of the medical instrument 41a to be connected to the connecting portion 42a is a rigid endoscope. Shaft-shaped medical instruments other than the rigid endoscope may be also connected to the connecting portion 42a. Hereinafter, the medical instrument 41a is also referred to as a rigid endoscope 41a. The rigid endoscope 41a is connected to the connecting portion 42a so that an angle between a longitudinal direction of the rigid endoscope 41a and a floor-side surface 17a of the distal portion 12a is a specific angle. The floor-side surface 17a is a portion (surface) of the distal portion 12a oriented toward a floor 80 during use (see FIG. 1 and FIG. 2).

The distal portion 12b includes the connecting portion 42b. The connecting portion 42b is a connection adapter to which a medical instrument 41b is to be connected. An instrument including an end effector at a distal side of a shaft-shaped member of the instrument is connected to the connecting portion 42b. An example of the medical instrument 41b is grasping forceps having a grasping part on the distal side of the shaft-shaped member. Hereinafter, the medical instrument 41b is also referred to as a surgical instrument 41b. The surgical instrument 41b is connected to the connecting portion 42b so that an angle between the floor-side surface 17b and a direction in which the shaft-shaped member of the surgical instrument 41b extends is a specific angle. The floor-side surface 17b is a portion (surface) of the distal portion 12b oriented toward the floor 80 during use (see FIG. 1).

The distal portion 12c includes the connecting portion 42c. The connecting portion 42c is a connection adapter to which a medical instrument 41c is to be connected. An instrument including an end effector at a distal side of a shaft-shaped member of the instrument is connected to the connecting portion 42c. An example of the medical instrument 41c is grasping forceps having a grasping part on a distal side of the shaft-shaped member. Hereinafter, the medical instrument 41c is also referred to as a surgical instrument 41c. The surgical instrument 41c is connected to the connecting portion 42c so that an angle between the floor-side surface 17c and a direction in which the shaft-shaped member of the surgical instrument 41c extends is a specific angle. The floor-side surface 17c is a portion (surface) of the distal portion 12c oriented toward the floor 80 during use (see FIG. 1).

The rigid endoscope 41a and the surgical instruments 41b, 41c are examples of medical instruments. Examples of medical instruments to be connected to the connecting portions 42b, 42c include not only grasping forceps, but also medical instruments including, at a distal side of a shaft-shaped member thereof, electric scalpels, surgical scissors, staplers and the like, which are commonly used in laparoscopic surgery.

The manipulator arm 13a operates in response to an operation signal so that the rigid endoscope 41a achieves a desired orientation, in a state in which a specific portion of the rigid endoscope 41a connected to the connecting portion 42a is arranged at a desired location of a patient 50. More specifically, the manipulator arm 13a operates in response to the operation signal to arrange the specific portion of the rigid endoscope 41a at the desired location on a body surface of the patient 50. Hereinafter, the above-described specific portion of the rigid endoscope 41a is also referred to as a pivot movement point 45. Herein, the desired location on the body surface includes a portion on the body surface of the patient 50, such as a skin of the patient 50, as well as a site inside a through-hole 53 for a trocar 52, which is pierced into the skin of the patient 50. That is, the desired location on the body surface includes a site corresponding to the body surface inside the through-hole 53 for the trocar 52 provided in the patient 50, as well as a site slightly shifted from the body surface to an inner side of the body.

Furthermore, the manipulator arm 13a operates in response to the operation signal so that the orientation of the rigid endoscope 41a, whose pivot movement point 45 is arranged at the desired location on the patient 50, achieves a desired orientation relative to the patient 50.

That is, the manipulator arm 13a is controlled to arrange the pivot movement point 45 at the desired location on the patient 50 and to change the orientation of the rigid endoscope 41a in response to the operation signal, in a state in which the pivot movement point 45 as its center of rotation is arranged at the desired location on the patient 50. In other words, the manipulator arm 13a is controlled to rotate the rigid endoscope 41a around the pivot movement point 45 as its center of rotation in a state in which the pivot movement point 45 is arranged at the desired location on the patient 50, so that a relative angle of the rigid endoscope 41a is the desired angle relative to the patient 50 in response to the operation signal.

Herein, as shown in FIG. 4, the pivot movement point 45 is a portion of the rigid endoscope 41a inside the through-hole 53 for the trocar 52 arranged in the abdomen of the patient 50 when the rigid endoscope 41a is in use. A relative position of the pivot movement point 45 in the rigid endoscope 41a is set in advance based on information concerning the rigid endoscope 41a, such as its length and application. As shown in FIG. 4, the pivot movement point 45 is set to a position on the rigid endoscope 41a that is located a specific distance D distally away from a portion of the rigid endoscope 41a connected to the connecting portion 42a. As long as a relative position of the pivot movement point 45 relative to the connecting portion 42a, the distal portion 12a, or the like can be recognized, the relative position may be set based on the other portions as a reference.

Hereinafter, a relative arrangement position of the pivot movement point 45 relative to the connecting portion 42a when the rigid endoscope 41a is connected to the connecting portion 42a is referred to as an arrangement position P1. In other words, a relative arrangement position, relative to the connecting portion 42a, of the pivot movement point 45 is to be arranged when the rigid endoscope 41a is connected to the connecting portion 42a is also referred to as the arrangement position P1.

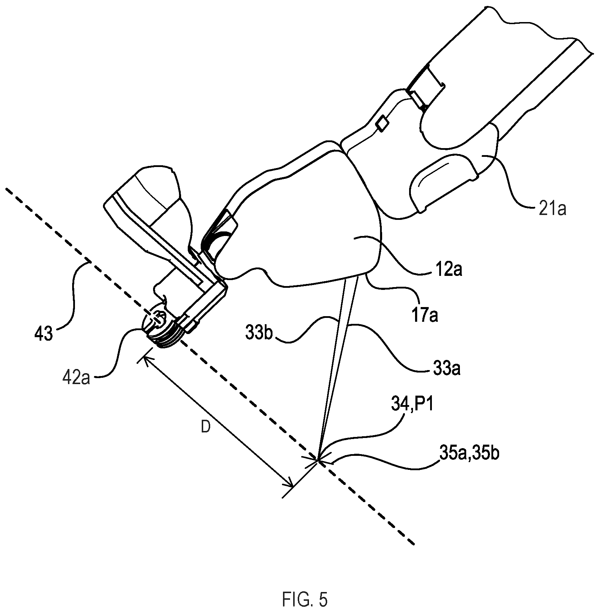

As shown in FIG. 4, the arrangement position P1 is set in advance to a position on an axis 43 that is located a distance D away in a direction extending from the connecting portion 42a toward the patient 50. Herein, the axis 43 is an axis that passes through the connecting portion 42a and that extends in the longitudinal direction of the rigid endoscope 41a through a center of a cross section perpendicular to the longitudinal direction of the rigid endoscope 41a connected to the connecting portion 42a (see FIGS. 4 and 5).

Similarly, the manipulator arm 13b is also controlled to arrange a specific portion on the shaft-shaped member of the surgical instrument 41b at the patient's desired location and to change the orientation of the surgical instrument 41b around the specific portion arranged at the patient's desired location as its center of rotation. Similarly, the manipulator arm 13c is also controlled to arrange a specific portion on the shaft-shaped member of the surgical instrument 41c at the patient's desired location and to change the orientation of the surgical instrument 41c around the specific portion arranged at the patient's desired location as its center of rotation.

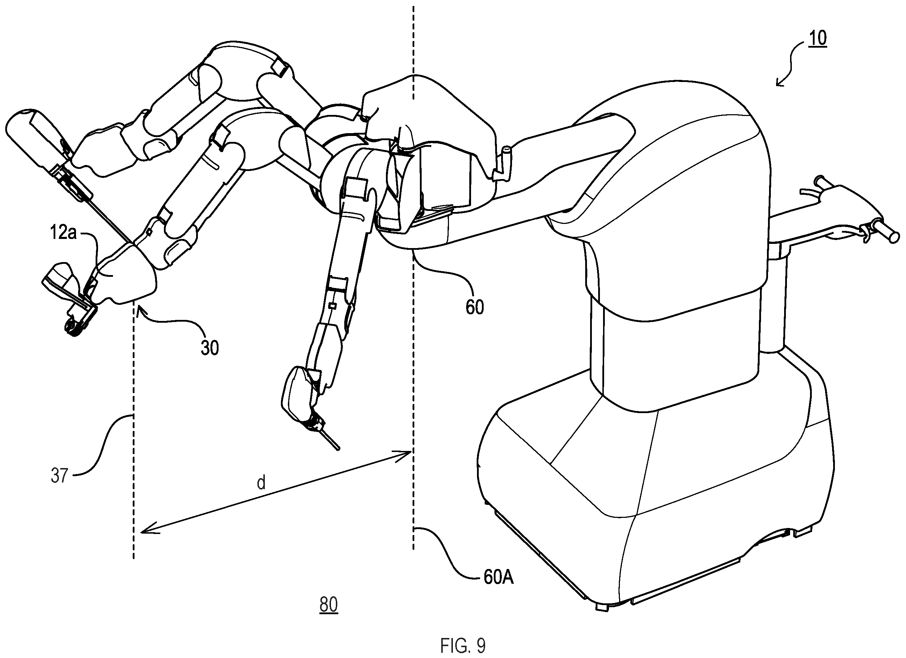

As shown in FIG. 1, the base portion 14 is connected to a column-shaped base support 15 extending from the cart portion 16. The base portion 14 is connected to the base support 15 so as to be rotatable around an axis 60A relative to the base support 15. As illustrated by broken lines in FIG. 1 and FIG. 9, the axis 60A is an axis extending in a direction perpendicular to the floor 80.

The cart portion 16 is a portion that supports the base portion 14 and the manipulator arms 13a, 13b, 13c via the base support 15. The worker can move the cart portion 16 by performing a specific operation. That is, the cart portion 16 can be moved to change the position of the medical device 10.

As shown in FIG. 2, the floor-side surface 17a of the distal portion 12a is provided with a first light emitter 30a. When the pivot movement point 45 is arranged at a desired location 54 on the patient 50, the first light emitter 30a emits light in a preset direction. This preset direction includes a direction extending from the first light emitter 30a toward the pivot movement point 45 of the rigid endoscope 41a connected to the connecting portion 42a. The first light emitter 30a emits the light toward the arrangement position P1. That is, when the pivot movement point 45 is arranged at the desired location 54 on the patient 50, the first light emitter 30a emits the light toward the arrangement position P1. When the pivot movement point 45 is arranged at the desired location 54, the first light emitter 30a emits the light toward the pivot movement point 45 (the arrangement position P1) arranged at the desired location on the patient 50.

The first light emitter 30a comprises a first light source 31a and a second light source 31b. The first light source 31a and the second light source 31b are aligned in a direction intersecting a direction extending from the base portion 14 toward the distal portion 12a. The direction extending from the base portion 14 toward the distal portion 12a is shown by an arrow denoted by X in FIG. 2.

The first light source 31a and the second light source 31b are arranged so that a first optical axis 33a of the first light source 31a and a second optical axis 33b of the second light source 31b intersect at a specific relative position from the first light source 31a and the second light source 31b. In other words, the first light source 31a and the second light source 31b are arranged such that the first optical axis 33a and the second optical axis 33b intersect at a position located a specific relative distance away from the first light source 31a and the second light source 31b.

As shown in FIG. 5, the first light source 31a and the second light source 31b are each arranged such that the first optical axis 33a and the second optical axis 33b intersect at a position on the axis 43 adjacent to the patient 50 by the distance D away from the connecting portion 42a. In other words, as shown in FIG. 5, the first light source 31a and the second light source 31b are each arranged such that the first optical axis 33a and the second optical axis 33b intersect at the arrangement position P1.

That is, the first light source 31a and the second light source 31b are each arranged such that the first optical axis 33a and the second optical axis 33b intersect at a position where the pivot movement point 45 is to be arranged, in a state in which the rigid endoscope 41a is connected to the connecting portion 42a.

The first light emitter 30a emits laser light having a complementary relationship color with red, which is the color of blood. That is, the first light source 31a and the second light source 31b each emit laser light having the complementary relationship color with red. Herein, the complementary color refers to colors located on opposite points of a straight line passing through a white point on a chromaticity diagram, with the white point in between. Alternatively, the complementary color refers to a color that is located directly opposite each other on a color wheel. The first light source 31a emits laser light of a blue-green color having a complementary relationship color with red. Similarly, the second light source 31b emits laser light of a blue-green having a complementary relationship color with red. The first light source 31a and the second light source 31b may each emit laser light having a color other than the blue-green color, as long as the colors are complementary relationship colors with red. Alternatively, the light other than laser light of any complementary relationship color with the color of blood may be emitted. The first light source 31a may emit light of the same color as the second light source 31b. The first light source 31a may emit light of a color different from the second light source 31b, as long as the color is a complementary relationship color with the color of blood.

The first light source 31a and the second light source 31b may be each configured to emit light of an opposite relationship color with blood. Herein, the "opposite color" refers to a color having properties opposite to a specific color in terms of hue, brightness, and saturation. For example, the first light source 31a and the second light source 31b may be configured to emit laser light of a color known as the opposite color to red, such as blue-green, yellow-green, blue, and blue-violet. Alternatively, light other than laser light of a color opposite to the color of blood may be emitted. The first light source 31a may be configured to emit light of the same color as that of the second light source 31b. Alternatively, the first light source 31a may emit light of a color different from that of the second light source 31b, as long as the color is an opposite relationship color with the color of blood.

The first light source 31a is configured such that projected light has a cross shape. Hereinafter, a projected image of the light from the first light source 31a is also referred to as a projected shape 35a. The second light source 31b is configured such that projected light has a cross shape. Hereinafter, a shape of the projected image of the light from the second light source 31b is also referred to as a projected shape 35b. As long as the worker can determine a site where the first optical axis 33a and the second optical axis 33b intersect, the first light source 31a and the second light source 31b may be configured such that either the projected shape 35a or the projected shape 35b has a shape other than the cross shape. Hereinafter, the site where the first optical axis 33a and the second optical axis 33b intersect is also referred to as an intersection point 44.

For example, the first light source 31a is configured such that the projected shape 35a has a cross shape, and the second light source 31b is configured such that the projected shape 35b has a shape other than the cross shape, for example, a circular shape. Alternatively, for example, the second light source 31b may be configured such that the projected shape 35b has a cross shape, and the first light source 31a may be configured such that the projected shape 35a has a shape other than the cross shape, for example, a circular shape. As long as the shape allows the worker to determine the intersection point 44, the first light source 31a and the second light source 31b may be configured such that the projected shape 35a and the projected shape 35b each have a shape other than the cross shape.

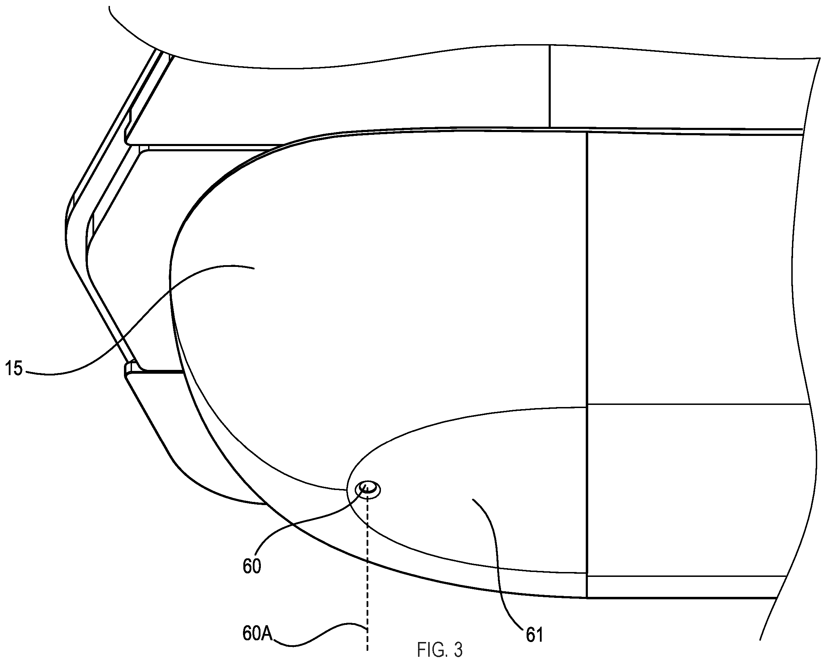

The base portion 14 is provided with a second light emitter 60 on a lower surface 61 oriented toward the floor 80 (see FIG. 1 and FIG. 3). The second light emitter 60 is a portion that emits red-purple laser light toward the floor 80. Herein, red-purple is a color that is complementary to green. In general, floors in surgery rooms are made of green flooring or are painted green. That is, the second light emitter 60 emits laser light of red-purple that is a complementary relationship color with the green floor 80. The second light emitter 60 may be a portion that emits laser light of other colors known to be a complementary relationship color with green. Alternatively, the second light emitter 60 may emit light other than laser light, which has a color known as the complementary color to green.

Alternatively, the second light emitter 60 may be a portion that emits laser light of a color known as having an opposite relationship color with green. For example, the second light emitter 60 may be a portion that emits light of a color, such as red-purple and red. Alternatively, the second light emitter 60 may emit light other than laser light of a color known as having an opposite relationship color with green.

The second light emitter 60 is situated on the axis 60A that passes through a center of rotation of the base portion 14 and that is perpendicular to the floor 80 (see FIG. 1 and FIG. 3). In other words, the second light emitter 60 is arranged directly below the center of rotation of the base portion 14.

In order to ensure an appropriate range of motion of the manipulator arm 13a, the controller 3 controls the manipulator arm 13a so that a horizontal distance between the second light emitter 60 and the first light emitter 30a is a specific distance. Specifically, as shown in FIG. 9, the controller 3 controls to change a position of the distal portion 12a so that a distance between the axis 60A and an axis 37 is a specific distance d, which is set in advance. The axis 37 is an axis that passes between the first light source 31a and the second light source 31b and that extends in the direction perpendicular to the floor 80.

That is, the controller 3 controls the manipulator arm 13a so that the manipulator arm 13a operates in response to the operation signal, in a state in which the horizontal distance between the second light emitter 60 and the first light emitter 30a is kept at the specific distance d.

### 2. Explanation of Setting Method

The following describes setting of the medical device 10 to be performed before starting laparoscopic surgery using a surgical robot system 1. Specifically, the following describes the setting performed on the manipulator arm 13a to which the rigid endoscope 41a is to be connected.

When the setting of the medical device 10 is performed, alignment between the medical device 10 and the patient 50 is performed in a state in which the rigid endoscope 41a is not initially connected to the connecting portion 42a. The worker performs a specific operation on the cart portion 16 to move the medical device 10 and arrange the medical device 10 near the patient 50. When arranging the medical device 10 near the patient 50, the worker arranges the medical device 10 at the desired location while checking a projection position of the light from the second light emitter 60 projected onto the floor 80.

For example, the worker moves the cart portion 16 while checking a distance between the projection position of the light from the second light emitter 60 projected onto the floor 80 and a patient bed (not shown) on which the patient 50 is placed, thereby obtaining a desired distance between the patient 50 and the medical device 10. Alternatively, if a positioning mark on the floor 80 is set in advance, the cart portion 16 is moved to allow the light from the second light emitter 60 to be projected toward the mark. As described above, since the manipulator arm 13a is controlled to move the distal portion 12a so that the distance between the second light emitter 60 and the first light emitter 30a is the specific distance d, the worker adjusts a distance between the patient 50 and the medical device 10, considering the specific distance d.

When the specific operation is performed and the medical device 10 is switched to the setting mode, the worker performs alignment between the patient and the medical device. Specifically, the worker performs alignment by manually changing the orientation of the manipulator arm 13a or by adjusting the position of the patient 50 while the arms 22a, 24a and the distal portion 12a are being held, so that the light from the first light emitter 30a is projected onto a surface at the desired location 54 on the patient 50. The desired location 54 is a portion of the abdomen of the patient 50 where the trocar 52 is arranged for surgery. Alternatively, in a state in which the trocar 52 is arranged on the patient 50, the desired location 54 is a portion of the patient 50 where the trocar 52 is arranged. The desired location 54 includes a site corresponding to the body surface of the patient 50 inside the through-hole 53 for the trocar 52 positioned on the patient 50.

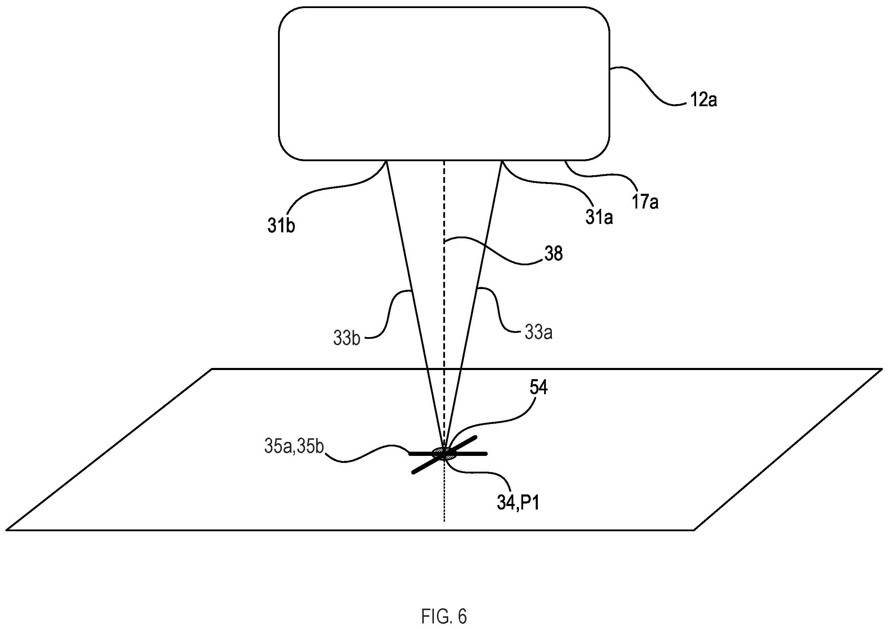

As shown in FIG. 6, when the position of the distal portion 12a and the position of the patient 50 are adjusted, the position of the distal portion 12a is adjusted and the position of the patient 50 is adjusted so that the shape 35a of the light from the first light source 31a and the shape 35b of the light from the second light source 31b are projected and overlap each other onto the surface at the desired location 54. That is, the worker performs the alignment so that the intersection point 34 of the first optical axis 33a and the second optical axis 33b is projected onto a surface at the desired location 54. The position of the distal portion 12a is adjusted and the position of the patient 50 is adjusted so that the cross-shaped center portions of the projected shapes 35a, 35b projected and overlap each other are projected onto the surface of the patient at the desired location 54.

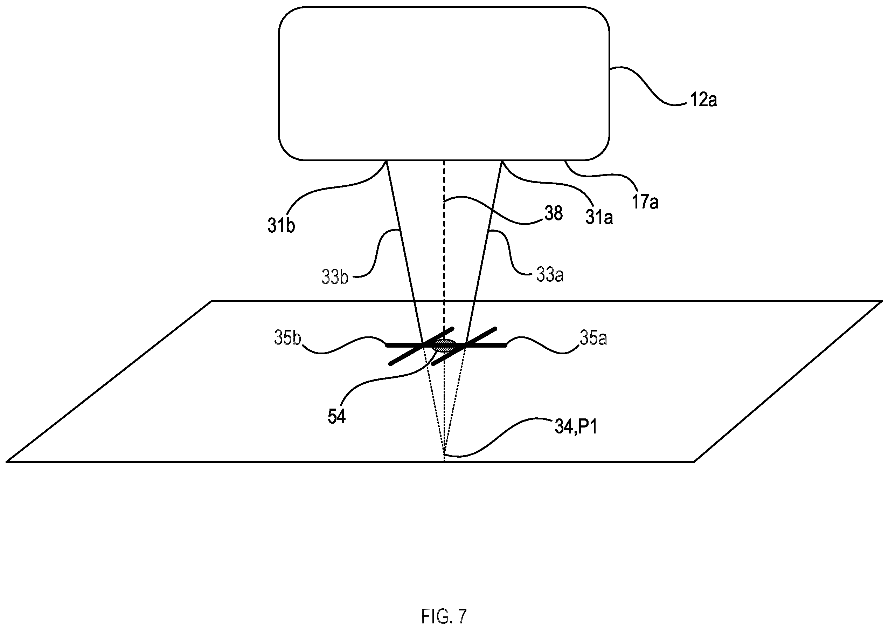

As shown in FIG. 7, when the desired location 54 is arranged closer to the distal portion 12a than the intersection point 34, the projected shape 35a and the projected shape 35b are at positions different apart from each other. That is, when the desired location 54 is arranged closer to the distal portion 12a than the arrangement position P1, the projected shape 35a and the projected shape 35b are at positions different apart from each other, as exemplified in FIG. 7.

**In** such a case, the position of the distal portion 12a and the position of the patient 50 are each adjusted so that a relative distance between the distal portion 12a and the desired location 54 is greater. Specifically, the position of the distal portion 12a is adjusted to be away from the patient or a height of the patient bed (not shown) is lowered, so that the relative distance between the distal portion 12a and the desired location 54 is adjusted.

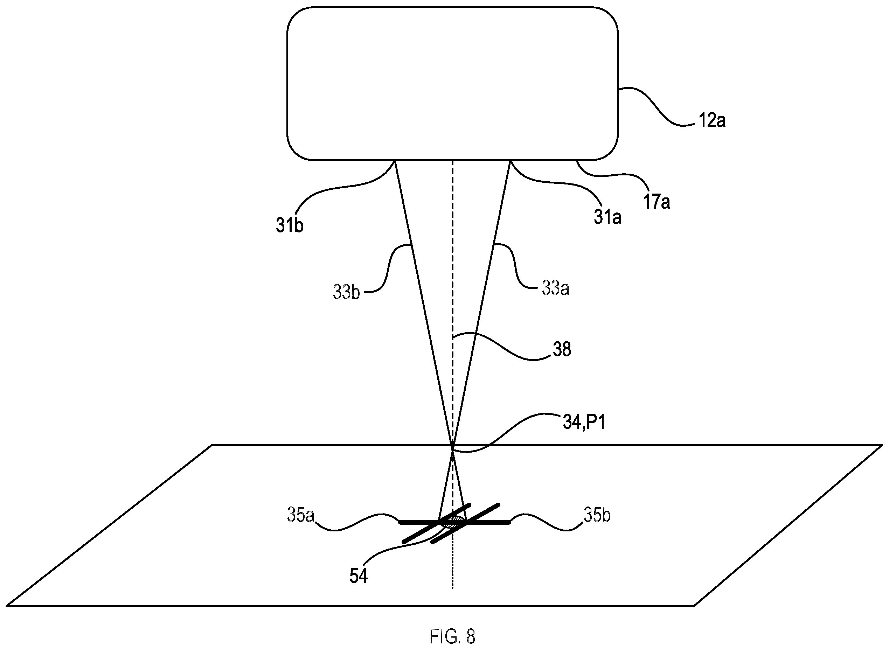

As shown in FIG. 8, when the desired location 54 is located farther from the distal portion 12a than the intersection point 34, the projected shape 35a and the projected shape 35b are projected so as to be at positions different apart from each other. That is, when the desired location 54 is located farther from the distal portion 12a than the arrangement position **P1,** the projected shape 35a and the projected shape 35b are projected so as to be at positions different apart from each other, as exemplified in FIG. 7.

**In** such a case, the position of the distal portion 12a and the position of the patient 50 are each adjusted so that the relative distance between the distal portion 12a and the desired location 54 is reduced. Specifically, the position of the distal portion 12a is adjusted to be closer to the patient, or the height of the patient bed (not shown) is increased, thereby adjusting the distance between the distal portion 12a and the desired location 54. That is, it is possible to accurately align the arrangement position P1 and the desired location 54 in a height direction by projecting the projected shape 35a and the projected shape 35b onto the desired location 54. In other words, it is possible to accurately align the arrangement position P1 and the desired location 54 in a light emission direction of the first light emitter 30a. Herein, it is to be stated that the light emission direction of the first light emitter 30a is a direction in which an axis 38 passes through the intersection point 34 and extends orthogonally to the floor-side surface 17a on a plane, which includes the first optical axis 33a and the second optical axis 33b (see FIGS. 6, 7, and 8).

When the manipulator arm 13a is set, the trocar 52 is arranged at the desired location on the patient 54. Specifically, the surgeon incises the abdomen of the patient 50 at the cross-shaped center portions of the projected shapes 35a, 35b that are projected and overlap each other at the desired location 54. The surgeon then inserts the trocar 52 into the incised abdomen of the patient 50 to be positioned.

The trocar 52 is a medical instrument used in laparoscopic surgery and the like, and is used to provide the through-hole 53 in a desired portion of the patient 50, passing from a skin to an abdominal cavity 51 through an abdominal wall of the patient 50.

When the trocar 52 is arranged on the patient 50, the rigid endoscope 41a is connected to the connecting portion 42a. As shown in FIG. 4, the distal side of the rigid endoscope 41a is connected to the connecting portion 42a in a state in which the rigid endoscope 41a is inserted into the through-hole 53 for the trocar 52 via the abdominal cavity 51. When the rigid endoscope 41a is connected to the connecting portion 42a, the position of the distal portion 12a is adjusted so that the through-hole 53 is arranged at the desired location 54 where the projected shape 35a and the projected shape 35b are projected and overlap each other, and then the rigid endoscope 41a is connected. When the rigid endoscope 41a is connected to the connecting portion 42a, the pivot movement point 45 of the rigid endoscope 41a is arranged inside the through-hole 53. That is, the pivot movement point 45 is arranged at the desired location 54.

### 3. Description of Intraoperative Operation

When an operation is performed on the operation unit 2 by the surgeon, the manipulator arm 13a performs movement in response to the operation signal from the controller 3. For example, when a specific operation on the operation unit 2 is performed by the surgeon, the manipulator arm 13a operates in response to the operation signal to change the orientation of the rigid endoscope 41a.

For example, when the surgeon operates the operation unit 2 in order to change a field of view of the rigid endoscope 41a, the orientation of the rigid endoscope 41a is changed so that the distal side of the rigid endoscope 41a points in the desired direction. That is, the manipulator arm 13a operates in response to the operation signal, thereby causing the rigid endoscope 41a to rotate around the pivot movement point 45 as a center of rotation with its distal side pointing in the desired direction. That is, the field of view of the rigid endoscope 41a is changed to a direction that is based on the operation performed by the surgeon.

According to the medical device 10 configured as described above, when the alignment is performed, the light is emitted from the first light emitter 30a in the preset direction. This makes it easier to perform a work of alignment between the medical device 10 and the patient 50. More specifically, the light is emitted from the first light emitter 30a toward the arrangement position P1 where the pivot movement point 45 is located when the rigid endoscope 41a is connected to the connecting portion 42a. This makes it easier to perform the work of alignment between the medical device 10 and the patient 50 so that the pivot movement point 45 (the arrangement position P1) and the desired location 54 on the patient are located at the same position.

The first light emitter 30a is situated on the distal portion 12a where the connecting portion 42a is provided. That is, the first light emitter 30a is arranged in the vicinity of the rigid endoscope 41a to be connected. This makes it easier to perform the work of alignment between the medical device 10 and the patient 50. In addition, since the first light emitter 30a is situated on the distal portion 12a, a portion of the manipulator arm 13a does not tend to be situated between the first light emitter 30a and the desired location 54 to block the light from the first light emitter 30a when the worker performs the alignment. Also, a portion of the body of the worker (assistant) who performs the alignment does not tend to be situated between the first light emitter 30a and the desired location 54, blocking the light from the first light emitter 30a. Furthermore, since the first light emitter 30a is situated on the distal portion 12a that is to be arranged in the vicinity of the desired location 54, even if an arrangement position or the like of the manipulator arm 13a is changed, the position of the light emitted from the first light emitter 30a does not tend to be significantly shifted. Also, even if the position of the light emitted from the first light emitter 30a is shifted, it is possible to easily modify a positional shift simply by modifying the position of the distal portion 12a. That is, the work of alignment between the patient and the medical device can be easily and efficiently performed. In addition, an accurate alignment can be performed.

By achieving accurate alignment, it is possible to inhibit unnecessary stress from being imposed on the patient when the orientation of the rigid endoscope 41a is changed. Due to lack of the accurate alignment, a portion of the rigid endoscope 41a inside the through-hole 53 for the trocar 52 is moved when the orientation of the rigid endoscope 41a is changed, thereby to cause unnecessary stress. This can be inhibited from being imposed on the patient.

The first light emitter 30a comprises the first light source 31a and the second light source 31b. The first light source 31a and the second light source 31b are arranged such that the first optical axis 33a and the second optical axis 33b intersect at a specific relative position from the first light source 31a and the second light source 31b. In other words, the first light source 31a and the second light source 31b are arranged such that the first optical axis 33a and the second optical axis 33b intersect at a position that is a specific distance away from each of the first light source 31a and the second light source 31b. This makes it easier to perform an accurate alignment in the light emission direction (height direction) of the first light emitter 30a. In addition, for example, when the first light source 31a and the second light source 31b are arranged such that the first optical axis 33a and the second optical axis 33b intersect at the arrangement position P1, it is possible to easily perform an accurate alignment between the desired location 54 on the patient 50 and the light emission direction (height direction) of the first light emitter 30a (height direction) at the pivot movement point 45.

The first light source 31a and the second light source 31b are aligned in a direction intersecting the direction extending from the base portion 14 toward the distal portion 12a. This makes it easier for the worker to perform the work of alignment when performing alignment, for example, by moving the distal portion 12a in a direction away from the base portion 14 or by moving the distal portion 12a in a direction closer to the base portion 14. That is, when adjusting the position by moving the distal portion 12a closer to or away from the base portion 14, it is easier to perform the work of alignment so that the projected shape 35a and the projected shape 35b are projected and overlap each other at the desired location 54.

The first light source 31a and the second light source 31b are configured such that at least one of the projected shape 35a or the projected shape 35b has a cross shape. Accordingly, in a case in which the alignment is not performed properly, the worker can determine the extent to which a position of the intersection point 34 and the position of the desired location 54 are misaligned, based on the cross-shaped projected images as a reference. Therefore, the worker can easily perform the work of alignment between the medical device 10 and the patient 50. Furthermore, when the first light source 31a and the second light source 31b are configured such that both the projected shape 35a and the projected shape 35b each have a cross shape, it is possible to easily determine the extent to which the position of the intersection point 34 and the position of the desired location 54 are misaligned. That is, the worker can easily perform the work of alignment between the medical device 10 and the patient 50.

The medical device 10 includes the cart portion 16, which is movable. Accordingly, the medical device 10 can be moved to a desired position. **In** addition, the arrangement position of the medical device 10 relative to the patient 50 can be adjusted.

Furthermore, the second light emitter 60 is provided on the lower surface 61 of the base portion 14. The light projected onto the floor 80 from the second light emitter 60 is checked, so that it is possible to easily grasp a distance between the medical device 10 and the patient 50. That is, it is easy to arrange the medical device 10 near the patient 50.

The manipulator arm 13a moves the position of the distal portion 12a so that a distance between the first light emitter 30a and the second light emitter 60 is a specific distance. Accordingly, the manipulator arm 13a and the position of the patient 50 can be aligned in a state in which the range of motion of the manipulator arm 13a is secured.

The first light source 31a emits light of a complementary color to red and/or a color opposite to red. That is, the first light source 31a and the second light source 31b each emit light of the complementary color to red, and/or the color opposite to red. The trocar 52 is placed in the patient's abdomen through an incision of the tissues. For this reason, at the site where the light from the first light source 31a is projected, the patient's blood is often present. Since the blood is red, light of the complementary color or the opposite color is easily distinguishable by the worker performing the alignment. This enables the worker to easily perform alignment between the manipulator arm 13a and the patient.

The second light emitter 60 emits light of the complementary color to green and/or the color opposite to green. In general, floors of surgery rooms where laparoscopic surgery is performed are green. Accordingly, when the light of the complementary color to green and/or the light of the color opposite to green are/is projected from the second light emitter 60, the worker performing the alignment can easily distinguish the light projected onto the floor 80, which is green, from the second light emitter 60. That is, the worker can easily perform alignment between the patient 50 and the medical device 10.

The technical scope according to the present disclosure is not limited to the above-described embodiments, and various modifications can be made without departing from the spirit according to the present disclosure.

For example, the first light emitter 30a may be provided on the distal portion 12b. In such a manner, the trocar 52 for inserting the surgical instrument 41b is inserted can be placed in an appropriate position. Similarly, the first light emitter 30a may be provided on the distal portion 12c.

Furthermore, the present disclosure may only need to be consistent with the spirit of the disclosure described in the above-described embodiments, and is not limited to the above-described embodiments. Therefore, at least two of the above-mentioned embodiments may be combined, or any of the configuration requirements illustrated or described with reference numerals in the above-mentioned embodiments may be omitted.

The present disclosure also provides, for example, the following items.

### (Item 1)

A medical device in which a pivot movement point of a medical instrument connected to the medical device is arranged at a desired location on a body surface of a patient, the medical device comprising at least:
a base portion;
a connecting portion to which the medical instrument is to be connected;
a distal portion including the connecting portion; and
a support portion connected to each of the base portion and to the distal portion and configured to change an orientation of the medical instrument to a desired orientation around the pivot movement point as a center of rotation, wherein
the distal portion further includes a first light emitter configured to emit light in a preset direction when the pivot movement point is arranged.

### (Item 2)

The medical device according to Item 1, wherein
the first light emitter includes:
   a first light source; and
   a second light source, and
the first light source and the second light source are arranged such that a first optical axis of the first light source and a second optical axis of the second light source intersect at a specific relative position from the first light source and the second light source.

### (Item 3)

The medical device according to Item 2, wherein
the first light source and the second light source are aligned in a direction intersecting a direction extending from the base toward the distal portion.

### (Item 4)

The medical device according to Item 2 or Item 3, wherein
at least one of the first light source or the second light source is configured such that a projected light has a cross shape.

### (Item 5)

The medical device according to any one of Item 1 through Item 4, further comprising:
a cart portion that is movable and to which the base portion is mounted, wherein
the base portion includes a second light emitter configured to emit light toward a floor where the cart portion is arranged.

### (Item 6)

The medical device according to Item 5, wherein
the support portion is configured to change a position of the distal portion so that a distance between the first light emitter and the second light emitter is a specific distance.

### (Item 7)

The medical device according to any one of Item 1 through Item 6, wherein
the first light emitter is configured to emit light of a color complementary to red and/or a color opposite to red.

### (Item 8)

The medical device according to any one of Item 5 through Item 7, wherein
the second light emitter is configured to emit light of a color complementary to green and/or a color opposite to green.

## Claims

1. A medical device in which a pivot movement point of a medical instrument connected to the medical device is arranged at a desired location on a body surface of a patient, the medical device comprising:
a base portion;
a connecting portion to which the medical instrument is to be connected;
a distal portion including the connecting portion; and
a support portion connected to each of the base portion and the distal portion and configured to change an orientation of the medical instrument to a desired orientation around the pivot movement point as a center of rotation, wherein
the distal portion further includes a first light emitter configured to emit light in a preset direction when the pivot movement point is arranged.

2. The medical device according to claim 1, wherein
the first light emitter includes:
a first light source; and
a second light source, and
the first light source and the second light source are arranged such that a first optical axis of the first light source and a second optical axis of the second light source intersect at a specific relative position from the first light source and the second light source.

3. The medical device according to claim 2, wherein
the first light source and the second light source are aligned in a direction intersecting a direction extending from the base portion toward the distal portion.

4. The medical device according to claim 2, wherein
at least one of the first light source or the second light source is configured such that a projected light has a cross shape.

5. The medical device according to any one of claim 1 through claim 4, further comprising:
a cart portion that is movable and to which the base portion is mounted, wherein
the base portion includes a second light emitter configured to emit light toward a floor where the cart portion is arranged.

6. The medical device according to claim 5, wherein
the support portion is configured to change a position of the distal portion such that a distance between the first light emitter and the second light emitter is a specific distance.

7. The medical device according to claim 1 or 2, wherein
the first light emitter is configured to emit light of a color complementary to red and/or a color opposite to red.

8. The medical device according to claim 5, wherein
the second light emitter is configured to emit light of a color complementary to green and/or a color opposite to green.
